# EUROPEAN PATENT SPECIFICATION

(11) **EP 1 673 136 B1**
(45) Date of publication and mention of the grant of the patent: **11.06.2008**
(21) Application number: 04769955.8
(22) Date of filing: 07.09.2004
(51) Int. Cl.: A61N 1/04

(54) **APPARATUS FOR PACKAGING AN ELECTRODE**
GERÄT ZUR VERPACKUNG EINER ELEKTRODE
APPAREIL POUR ENCAPSULER UNE ELECTRODE

(30) Priority: 06.10.2003 US 509011 P
(43) Date of publication of application: 28.06.2006
(73) Proprietor: Koninklijke Philips Electronics N.V., 5621 BA Eindhoven (NL)
(72) Inventor: JONSEN, Eric, Bothell, WA 98041-3003 (US)
(74) Representative: Schouten, Marcus Maria
(86) International application number: PCT/IB2004/051705
(87) International publication number: WO 2005/032648

(56) References cited:
- WO-A-03/037176
- US-A- 5 402 884
- US-A- 6 101 413

## Description

Aspects of this invention relate generally to electrode storage, and more particularly to an apparatus and method for packaging a medical electrode, and to an electrotherapy device having an electrode.

Modern medical electrode packaging provides a sealed electrode storage environment and through-wall electrical connectivity to electrotherapy devices such as external defibrillators. Most modem electrodes use a water-based gel to adhere the electrode to the skin and to provide a good conductive path between skin and electrode. Because the hydrogels tend to gradually dry out and become ineffective, sealing of the electrode from the environment extends the shelf-life of the electrode significantly. Thus, electrode packaging is typically either a flexible, heat-sealable laminate material, or a rigid, molded plastic material, which serves as a moisture barrier.

Flexible housings provide economical, simple packaging for electrodes in many instances. Electrode wiresets extend through exteriors of known flexible housings, and connect directly to electrotherapy devices. Electrode function or sterility, however, may be compromised when electrode wiresets protrude through flexible housings--for example, flexing may weaken the bond between the electrode wireset and the flexible material, and/or the flexible material may remain adhered to electrode wiresets after placement of electrodes on a patient, causing user confusion or delay. Adequately sealing areas where electrode wiresets extending through flexible housings may increase manufacturing costs or complexity.

Rigid structures offer an alternative to flexible housings. Walls of rigid structures may include insert-molded electrical contacts, such as pins, which provide through-wall electrical connectivity between enclosed electrode wiresets and external electrotherapy devices. Although rigid housing structures may sometimes be more expensive and have higher manufacturing costs than flexible housings, rigid structures are often selected because they have been designed to enclose electrode wiresets without compromising the structures' seals, and they offer simple user interfaces. Rigid structures, however, may be less desirable in some applications at high altitudes, when pressures inside the structures greatly exceed ambient pressures, and heat-seal film, which is often stretched over structure openings, may be vulnerable to puncture.

From WO 03/037176 A1 an enclosure for a medical electrode which seals the medical electrode against moisture loss while the medical electrode remains in electrical communication with a medical instrument is known. This enclosure comprises a wall of flexible material and a connector in this wall, wherein the connector is adapted to connect to the medical instrument and to the medical electrode, respectively.

Further, from US 5,402,884 a sealed package system for housing at least one medical electrode apparatus and for enabling periodic testing thereof is known. The package comprises a thin, generally flat flexible envelope constructed and arranged to form an interior cavity for enclosing a conductive gel contact surface of an electrode apparatus-The envelope has one continuous layer of a homogeneous, non-conductive, polymeric material. Further, a structure for conducting current across the envelope to the interior cavity is provided, wherein the conductive structure is electrically connectible to the electrode conductive contact surface.

It is the object of the invention to provide a resistant and robust enclosure which does not run the risk of being damaged when connecting a medical instrument.

This object is addressed by the subject matter of claim 1. Preferred embodiments are described in the dependent claims.

There is therefore a need for an apparatus and method for packaging a medical electrode, which is adapted for use with a heat-sealable flexible enclosure material, which encloses the electrode wireset without compromising the enclosure seal, which enables the electrode to remain responsive to an electrotherapy device during storage and patient use, and which has a simple user interface.

In accordance with one aspect of the present invention, an apparatus for packaging a medical electrode having an electrode wireset is provided. The apparatus comprises a flexible electrode wireset package having an interior connector on the inside of the package which is connected through the package to an exterior connector on the outside of the package. An electrode wireset can be sealed in the package with the electrode connector connected to the interior connector in the inside of the package. When the exterior connector on the outside of the package is connected to an instrument such as a patient monitor or electrotherapy device, the electrode wireset which is sealed inside the package is in electrical communication with the instrument for testing or other analysis. When the electrode is put to use, the package is opened and the electrode can be immediately applied to the patient with no change in connections. That is, the electrode can remain connected to the instrument through the package connectors while in use. Alternatively, the electrode wireset can be unplugged from the interior package connector and plugged directly into the instrument, with the package placed away from the treatment area.

A further benefit of the present invention is that the packaged electrode can be removed from the instrument and replaced with another electrode without the need to open the sealed package. Another benefit is that the electrode can be used on a different instrument by unplugging the exterior connector from the instrument and reconnecting it to a different instrument. Yet another benefit is the ability to open the package and unplug and remove the electrode from the package, then be able to immediately use the electrode with a different instrument if desired.

In a described embodiment the interior and exterior connectors are attached to and electrically connected through a rigid plastic flange which can be readily heat-sealed in an opening in the wall of a flexible pouch package. This embodiment obviates the need to try to seal the seam of the package around the wires or connector of an electrode, which is prone to leakage and seal failure.
FIG. 1 illustrates a medical electrode usable in connection with aspects of the present invention.
FIG. 2 is a schematic block diagram of an external defibrillator system usable in connection with the medical electrode of FIG. 1, and in connection with aspects of the present invention.
FIG. 3 is a partial perspective view of an apparatus for packaging a medical electrode in accordance with aspects of the present invention.
FIG. 4 is cross-sectional side view of the apparatus shown in FIG. 3.
FIG. 5 depicts the apparatus shown in FIGs. 3 and 4 in use with a heat-sealable flexible material, in accordance with aspects of the present invention.

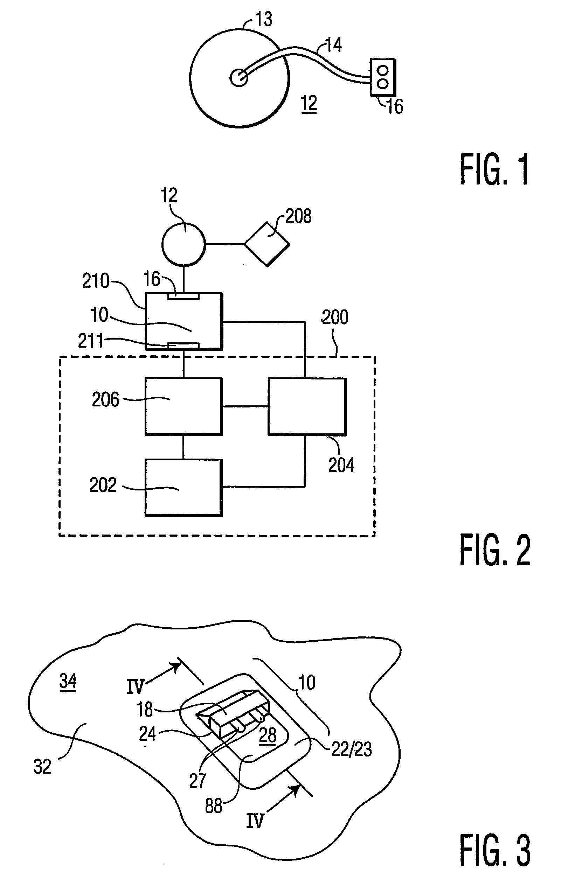

Turning now to the drawings, wherein like numerals designate like components, FIG. 1 illustrates basic functional elements of a medical electrode 12, which may be packaged in accordance with certain aspects of the present invention. Electrode 12 includes a patient portion 13, a wireset 14 coupled to patient portion 13, and a detachably connectable electrode adapter 16 coupled to wireset 14. Electrode 12 may be used with an electrotherapy system, such as an automatic or manual external defibrillator system, or another type of electrotherapy system, such as pacing or cardiac monitoring systems.

A schematic block diagram of an external defibrillator system 200 that uses electrode 12 is depicted in FIG. 2. Defibrillator system 200 includes an energy source 202, which provides voltage or current pulses. A controller 204 operates an energy delivery system 206 to selectively connect and disconnect energy source 202 to and from one or more electrodes 12 electrically attachable to a patient 208. Defibrillator system 200 may further include elements (not shown) such as user input and/or output devices or displays, memories, or computer-executable instructions implemented in software, firmware, hardware or a combination thereof.

A signal path between defibrillator 200 and electrode 12 is provided via electrode interface 210, which may form part of, or be separate from, defibrillator system 200. Electrode interface 210 includes, at least in part, areas for receiving electrode adapter 16 and an energy delivery connector 211 (which may be, for example, an electrical plug), and further includes apparatus 10 configured in accordance with aspects of the present invention (discussed further below).

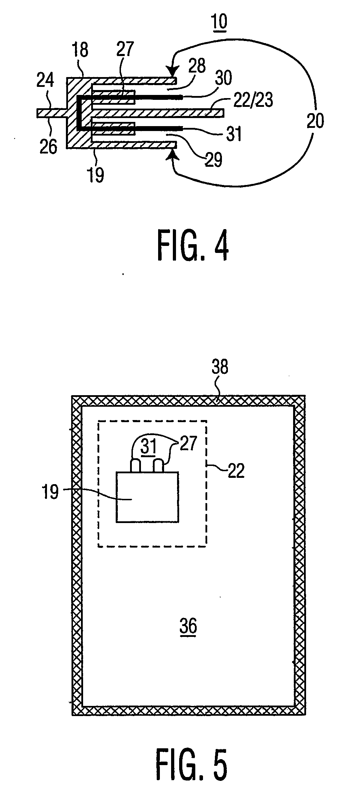

Apparatus 10 is shown in partial perspective view in FIG. 3, and in more detail in cross-sectional side view along line IV-IV in FIG. 4. Referring to FIG. 3, apparatus 10 is adapted for use with a heat-sealable flexible material 32, which has an interior side 34 (shown in cut-away view in FIG. 3) and an exterior side 36 (not visible; shown in FIG. 5 and discussed further below.) Material 32 is preferably arranged in laminate form, having at least three layers. A top layer is comprised of a non-conductive material with good printability such as polypropylene. A second layer is comprised of a moisture barrier, such as aluminum foil and/or low-density polyethylene. A bottom layer is comprised of a heat-sealable material such as polyethylene. A heat-sealable flexible laminate material 32 suitable for use with apparatus 10 is commercially available from Cadillac Products, Incorporated in Troy Michigan. Material 32 could alternatively be comprised of two layers, including a moisture barrier layer and a heat-sealing layer. The layers may also be arranged in a different order.

Referring to FIG. 4, apparatus 10 includes a housing 20 having two sides. A first housing side 18 defines an electrode connection region 28, which is sized to receive at least a portion of electrode 12, such as electrode adapter 16. A second housing side 19 defines a medical device connection region 29, which is sized to receive at least a part of a medical device, such as energy delivery connector 211 from defibrillator system 200 (shown in FIG. 2). As shown, housing sides 18, 19 have partially open polyhedrous configurations, although housing sides 18 and 19 may assume any desired geometric configurations. Housing 20 is preferably an integral, molded plastic device, but may be formed of a plurality of interconnected parts of any suitable material.

A sealing surface 22 having two sides 24, 26 is coupled to housing 20. As shown, sealing surface side 24 is coupled to first housing side 18, and sealing surface side 26 is coupled to second housing side 19. Sealing surface 22 also has an attachment area 23, for attaching sealing surface 22 to one or more laminate layers of heat-sealable flexible material 32 (shown in FIG. 3). Attachment area 23 preferably extends outside of a perimeter of housing 20, but may be interposed between housing sides 18, 19. Sealing surface 22 is preferably a planar plastic flange, integrally formed with housing 20, but may be constructed separately from housing 20 and attached thereto in any suitable fashion.

Referring now to both FIG. 3 and FIG. 4, at least one signal conductor 27 having one end 30 arranged in electrode connection region 18, and another end 31 arranged in medical device connection region 31, is operable to provide a signal path traversing heat-sealable flexible material 32. Signal conductor 27 is preferably an electrical conductor, such as a pin connector, insert-molded into housing 20, but may be any device now known or later developed capable of causing electrical signals, or representations thereof, to traverse heat-sealable flexible material 32. Signal conductor 27 may be disposed in housing sides 18, 19, heat-sealable flexible material 32, and/or sealing surface 22.

FIG. 5 depicts apparatus 10 in use with heat-sealable flexible material 32, in accordance with certain aspects of the present invention. As shown, side 19 of housing 20 is disposed on exterior 36 of heat-sealable flexible material 32. Two signal conductors 27 are arranged in medical device connection region 31 (partially visible). Housing side 18 (not visible) is disposed on interior 34 (also not visible) of heat-sealable flexible material 32.

Apparatus 10 is preferably inserted into a keyed, die-cut hole in material 32, and housing 20 is preferably attached to material 32 by heat-sealing sealing surface 22 to one or more surface(s) of laminates defining interior 34 and/or exterior 36 of material 32. Sealing surface 22, however, may also be attached in other way-by application of hot-melt adhesive or double-sided adhesive tape, or ultrasonic welding, for example. A zone 38, preferably on a perimeter of material 32, is heat-sealable to form a container for storage of electrode 12. For example, material 32 may be folded and sealed at zone 38, to form a sealed pouch-type container, with interior 34 forming the inside of the container Apparatus 10 may be automatically inserted in material 32 before pouches are formed, at the sheet-stock stage, or may be installed after storage pouches or containers are formed from material 32.

After housing 20 has been attached to heat-sealable flexible material 32, and after at least one signal conductor 27 having ends 30, 31 is arranged in electrode connection region 18 and medical device connection region 31, respectively, to provide a signal path traversing material 32, then one or more electrodes 12 may be arranged in electrode connection region 18, in such a manner as to be responsive to end 30. For example, when signal conductor 27 is an electrical connector, such as a pin, electrode adapter 16 may be detachably coupled to the pin. Patient portion 13 and wireset 14 are preferably disposed relative to side 34 of material 32-preferably enclosed in a container formed at least in part by interior 34. The container may be hermetically sealed for electrode storage or transport.

Communication between defibrillator 200 and packaged electrode 12 may be established via apparatus 10 when a portion of a medical device, such as energy delivery connector 211, is arranged in medical device connection region 19 and coupled to one more signal conductors 27. Thus, when an electrode 12 is packaged in accordance with aspects of the present invention, and when the medical device is coupled to signal conductors in region 31, in such a manner that the signal path traversing material 32 is operational, electrode 12 remains responsive to the medical device during shipping, storage or use.

For example, communication may be established between packaged electrode 12 and a medical device for purposes of testing the functionality of electrode 12 while the medical device is in standby mode, and the medical device and/or electrode 12 may be enabled to alarm if a problem is detected.

In another example, communication between packaged electrode 12 and the medical device may be used to monitor other parameters of interest, such as interior environmental conditions of electrode 12, or packaging thereof. Additional connections, such as signal conductors 27, could be used to facilitate monitoring such additional parameters.

In a further example, apparatus 10 may provide communication between the medical device and packaged electrode 12 during normal operation of the medical device in connection with patient therapy. In the case of defibrillator 200 (shown in FIG. 2), electrode 12 may be placed patient 208 without detachment of electrode adapter 16 from apparatus 10, and defibrillator 200, may continue to provide/receive electrical communication and information to/from electrode 12 via apparatus 10 during patient treatment and monitoring, without further user intervention.

Continuing to refer to FIG. 2, in a still further example, when electrode adapter 16 is detached from apparatus 10, electrode adapter 16 may be re-attached to apparatus 10, or may be attached directly to another device, such as another electrotherapy device (not shown) for use in care of patient 208.

Thus, apparatus 10 provides flexible manufacturing/assembly, a simple user interface that reduces set-up time to a minimum, and usability with heat-sealable flexible materials that enclose electrode wiresets without compromising enclosure seals. Electrodes packaged using apparatus 10 remain responsive to electrotherapy devices, and may be monitored for functionality and used for patient therapy without interruption of connection from the electrotherapy device.

The embodiment(s) depicted and described herein are meant to be illustrative in nature, and it will be understood that housings of any shapes and sizes may be designed using the principles set forth herein, and used for various commercial and consumer applications. It will also be understood that aspects of the invention are not limited to the specific embodiments described above, that other and further forms of the invention may be devised without departing from the and scope of the appended claims and their equivalents, and that aspects described and claimed herein are to be accorded the widest scope consistent with the principles and features disclosed herein.

## Claims

1. An enclosure for a medical electrode (12) which seals the medical electrode (12) against moisture loss while the medical electrode (12) remains in electrical communication with a medical instrument (200) comprising:
a wall of flexible material (32) forming an enclosure which is adapted to be sealed against moisture loss ;
an interior connector (30) located on the interior (34) of the wall of flexible material (32) and adapted to connect to the medical electrode (12); and
an exterior connector (31) located on the outside (36) of the wall of flexible material (32) and adapted to connect to the medical instrument (200), the exterior connector (31) being in electrical communication with the interior connector (30),
**characterized in that**
a flange (22) is provided having the interior connector (30) and the exterior connector (31) located on opposite sides thereof,
the interior connector (30) and the exterior connector (31) are sealed through a hole in the wall of flexible material;
the flange (22) is sealed to the hole in the wall of flexible material (32), and
the flange (22) is formed of a rigid insulative material.

2. The enclosure according to claim 1, wherein the medical electrode (12) comprises a wireset (14) which is coupled to the interior connector (30), wherein the medical electrode (12) is sealed inside of the enclosure.

3. The enclosure according to claim 2, wherein the exterior connector (31) is adapted for coupling the medical instrument (200) in electrical communication with the medical electrode (12).

4. The enclosure according to any of claims 1 to 3, wherein the flange (22) is formed of a heat-sealable material,

5. The enclosure of claim 4, wherein the flange (22) is heat-sealed to the periphery of the hole in the wall of flexible material (32).

6. The enclosure according to any of claims 1 to 5, wherein the wall of flexible material (12) comprises a hermetically sealable pouch for storing the medical electrode (12).

7. The enclosure according to any of claims 1 to 6, wherein the interior connector (30) and the exterior connector (31) comprise an electrical connector having the first end disposed in the interior of the enclosure, and a second end disposed on the exterior of the enclosure.

## Patentansprüche

1. Gehäuse für eine medizinische Elektrode (12), das die medizinische Elektrode (12) gegen Feuchtigkeitsverlust abdichtet, während die medizinische Elektrode (12) in elektrischer Kommunikation mit einem medizinischen Instrument (200) bleibt, wobei das Gehäuse Folgendes umfasst:
eine Wand aus biegbarem Material (32), die ein Gehäuse bildet und dafür vorgesehen ist, gegen Feuchtigkeitsverlust abzudichten;
einen internen Steckverbinder (30), der sich auf der Innenseite (34) der Wand aus biegbarem Material (32) befindet und für die Verbindung mit der medizinischen Elektrode (12) ausgelegt ist; und
einen externen Steckverbinder (31), der sich auf der Außenseite (36) der Wand aus biegbarem Material (32) befindet und für die Verbindung mit dem medizinischen Instrument (200) vorgesehen ist, wobei der externe Steckverbinder (31) in elektrischer Kommunikation mit dem internen Steckverbinder (30) steht,
**dadurch gekennzeichnet, dass**
ein Flansch (22) vorgesehen ist, bei dem der interne Steckverbinder (30) und der externe Steckverbinder (31) auf gegenüberliegenden Seiten angeordnet sind,
der interne Steckverbinder (30) und der externe Steckverbinder (31) durch ein Loch in der Wand aus biegbarem Material versiegelt sind:
der Flansch (22) mit dem Loch in der Wand aus biegbarem Material (32) versiegelt ist, und
der Flansch (22) aus einem starren, isolierenden Material gebildet ist.

2. Gehäuse nach Anspruch 1, wobei die medizinische Elektrode (12) einen Kabelsatz (14) umfasst, der mit dem internen Steckverbinder (30) gekoppelt ist, wobei die medizinische Elektrode (12) im Inneren des Gehäuses versiegelt ist.

3. Gehäuse nach Anspruch 2, wobei der externe Steckverbinder (31) zur Kopplung des medizinischen Instruments (200) in elektrischer Verbindung mit der medizinischen Elektrode (12) vorgesehen ist.

4. Gehäuse nach einem der Ansprüche 1 bis 3, wobei der Flansch (22) aus Heißsiegelmaterial gebildet ist.

5. Gehäuse nach Anspruch 4, wobei der Flansch (22) mit der Peripherie des Lochs in der Wand aus biegbarem Material (32) heißversiegelt ist.

6. Gehäuse nach einem der Ansprüche 1 bis 5, wobei die Wand aus biegbarem Material (12) eine hermetisch versiegelbare Tasche zur Aufbewahrung der medizinischen Elektrode (12) umfasst.

7. Gehäuse nach einem der Ansprüche 1 bis 6, wobei der interne Steckverbinder (30) und der externe Steckverbinder (31) einen elektrischen Steckverbinder umfassen, dessen erstes Ende im Inneren des Gehäuses angeordnet ist und dessen zweites Ende auf der Außenseite des Gehäuses angeordnet ist.

## Revendications

1. Enceinte pour une électrode médicale (12) qui scelle l'électrode médicale (12) contre la perte d'humidité alors que l'électrode médicale (12) reste en communication électrique avec un instrument médical (200) comprenant:
une paroi constituée de matériau flexible (32) formant une enceinte qui est adaptée de manière à être scellée contre la perte d'humidité;
un connecteur intérieur (30) qui se situe du côté intérieur (34) de la paroi constituée de matériau flexible (32) et qui est adapté de manière à être connecté à l'électrode médicale (12); et
un connecteur extérieur (31) qui se situe du côté extérieur (36) de la paroi constituée de matériau flexible (32) et qui est adapté de manière à être connecté à l'instrument médical (200), le connecteur extérieur (31) étant en communication électrique avec le connecteur intérieur (30),
**caractérisée en ce que**
il est fourni une bride (22) ayant le connecteur intérieur (30) et le connecteur extérieur (31) positionnés sur les côtés opposés de celle-ci,
le connecteur intérieur (30) et le connecteur extérieur (31) sont scellés à travers un trou dans la paroi constituée de matériau flexible;
la bride (22) est scellée au trou dans la paroi constituée de matériau flexible (32), et
la bride (22) est formée à partir d'un matériau isolant rigide.

2. Enceinte selon la revendication 1, dans laquelle l'électrode médicale (12) comprend un jeu de fils (14) qui est couplé au connecteur intérieur (30) dans lequel l'électrode médicale (12) est scellée à l'intérieur de l'enceinte.

3. Enceinte selon la revendication 2, dans laquelle le connecteur extérieur (31) est adapté de manière à coupler l'instrument médical (200) en communication électrique avec l'électrode médicale (12).

4. Enceinte selon l'une quelconque des revendications précédentes 1 à 3, dans laquelle la bride (22) est formée à partir d'un matériau susceptible d'être scellé à chaud.

5. Enceinte selon la revendication 4, dans laquelle la bride (22) est scellée à chaud à la périphérie du trou dans la paroi constituée de matériau flexible (32).

6. Enceinte selon l'une quelconque des revendications précédentes 1 à 5, dans laquelle la paroi constituée de matériau flexible (12) comprend une pochette susceptible d'être scellée hermétiquement pour stocker l'électrode médicale (12).

7. Enceinte selon l'une quelconque des revendications précédentes 1 à 6, dans laquelle le connecteur intérieur (30) et le connecteur extérieur (31) comprennent un connecteur électrique ayant la première extrémité disposée dans l'intérieur de l'enceinte et ayant une deuxième extrémité disposée du côté extérieur de l'enceinte.
